Europäisches Patentamt

European Patent Office

Office européen des brevets

(11) Publication number: 0 247 860 B1

# EUROPEAN PATENT SPECIFICATION

(45) Date of publication of patent specification: 08.07.92  (51) Int. Cl.5: A61K 37/02, C07K 17/08

(21) Application number: 87304703.9

(22) Date of filing: 27.05.87

(54) Tumor necrosis factor formulation and its preparation.

(30) Priority: 29.05.86 US 868766

(43) Date of publication of application:
02.12.87 Bulletin 87/49

(45) Publication of the grant of the patent:
08.07.92 Bulletin 92/28

(84) Designated Contracting States:
AT BE CH DE ES FR GB GR IT LI LU NL SE

(56) References cited:
EP-A- 0 098 110
EP-A- 0 154 316
WO-A-87/00056
US-A- 4 179 337

CHEMICAL ABSTRACTS, vol. 101, no. 15, 8th
October 1984, page 29, abstract no. 122679c,
Columbus, Ohio, US; A. ABUCHOWSKI et al.:
"Cancer therapy with chemically modified
enzymes. I. Antitumor properties of polyeth-
ylene glycol-asparaginase conjugates", &
CANCER BIOCHEM. BIOPHYS. 1984, 7(2),
175-86

(73) Proprietor: CETUS CORPORATION
1400 Fifty-Third Street
Emeryville California 94608(US)

(72) Inventor: Katre, Nandini
6107 Jordan Avenue
El Cerrito California 94530(US)
Inventor: Thomson, James William
811 Talbot Avenue
Albany California 94706(US)
Inventor: Knauf, Michael James
2391 Princeton Drive
San Bruno California 94066(US)

(74) Representative: Bizley, Richard Edward et al
HEPWORTH LAWRENCE BRYER & BIZLEY
2nd Floor Gate House South West Gate
Harlow, Essex CM20 1JN(GB)

**Description**

This invention relates to a chemical modification of tumor necrosis factor (TNF) which alters and improves the chemical and/or physiological properties thereof.

Human TNF is a 157 amino acid protein containing two cysteine residues, one at position 69, and the other at position 101. TNF was first reported by Carswell et al., Proc. Natl. Acad. Sci. (USA) (1975), 72:3666, and has been shown to be cytotoxic selectively to neoplastic cells. TNF has been purified from cell culture by Matthews et al., Brit. J. Cancer (1981), 44:418 (from mononuclear phagocytes derived from BCG-injected rabbits) and by Mannel et al., Infect. Immun. (1980), 30:523; ibid. (1981), 33:156 (from cultures of macrophage-enriched peritoneal exudate cells from BCG-infected mice). The sequence encoding TNF produced by the human promyelocytic leukemia cell line (HL-60, ATCC Deposit No. CCL240) has been cloned and expressed in E. coli. See copending PCT US85/01921 filed October 3, 1985, published April 24, 1986 and PCT US86/02381.

The use of polypeptides in circulatory systems for the purpose of producing a particular physiological response is well known in the medicinal arts. A limitation to the potential therapeutic benefit derived from the clinical use of polypeptides is their ability to elicit an immune response in the circulatory system. This immune response may be caused by aggregates in the material prior to injection as described by R. Illig (1970), J. Clin. Endrocr., 31, 679-688, W. Moore (1978), J. Clin. Endrocrinol. Metab., 46, 20-27 and W. Moore and P. Leppert (1980), J. Clin. Endrocrinol. Metab., 51, 691-697. This response involves the production of antibodies to the polypeptides by the circulatory system into which they are injected. This antibody production may decrease or eliminate the desired biological function of the polypeptide, sometimes by causing reduced residence time in the circulatory system (reduced half-life) or by modifying the molecule by virtue of the antibody-polypeptide interaction.

Modification of these potentially useful therapeutic polypeptides so as to preclude or at least reduce an immune response while still maintaining desired physiological activities of the polypeptide would allow the use of these polypeptides in the mammalian circulatory system without the aforementioned disadvantages. In addition, due to the increased half-life of the circulating polypeptide, smaller amounts of the polypeptide would be required for the desired therapeutic effect than have heretofore been possible.

The problems of immunogenicity and short half-life in circulation set forth hereinabove and other undesirable properties of certain proteins are well recognized and various modifications of polypeptides have been undertaken to solve them. These include the modification of proteins with substantially straight chain polymers such as polyethylene glycol (PEG) or polypropylene glycol (PPG). For example, U.S. Patent No. 4,261,973 describes conjugation of immunogenic allergen molecules with non-immunogenic water-soluble polymers such as PEG to reduce the immunogenicity of the allergen. U.S. Patent No. 4,301,144 describes conjugation of hemoglobin to PEG, PPG, a copolymer of ethylene glycol with propylene glycol, or ethers, esters or dehydrated products of such polymers to increase the oxygen-carrying ability of the hemoglobin molecule. European Patent Publication 98,110, published January 11, 1984, discloses that coupling of a polypeptide or glycoprotein to a polyoxyethylene-polyoxypropylene copolymer increases the length of its physiological activity. Preferably the polypeptide or glycoprotein is an enzyme or native interferon, which are water soluble. U.S. Patent No. 4,179,337 discloses coupling of water-soluble polypeptides such as enzymes and insulin to PEG or PPG to reduce the immunogenicity of the polypeptide while retaining a substantial proportion of its desired physiological activity. U.S. Patent No. 4,002,531 discloses a different method of coupling enzymes to PEG through an aldehyde derivative.

U.S. Patent 4,055,635 discloses pharmaceutical compositions comprising a water-soluble complex of a proteolytic enzyme linked covalently to a polymeric substance such as polysaccharides.

U.S. Patent 3,960,830 discloses peptides bound to a polyalkylene glycol polymer such as polyethylene glycol.

U.S. Patent 4,088,538 discloses a reversibly soluble enzymatically active polymer enzyme product comprising an enzyme covalently bonded to an organic polymer such as polyethylene glycol.

U.S. Patent 4,415,665 discloses a method of coupling an organic ligand containing at least one primary or secondary amino group, at least one thiol group and/or at least one aromatic hydroxy group (described in col. 3, lines 19-36) to a polymeric carrier with at least one hydroxyl group (described in col. 2, lines 42-66).

U.S. Patent 4,495,285 discloses a non-immunogenic plasminogen activator, the amino acid side chains of which are coupled to a polyalkylene glycol through a coupling agent.

U.S. Patent 4,412,989 discloses an oxygen-carrying material containing hemoglobin or a derivative thereof covalently coupled through an amide bond to polyethylene or polypropylene glycol.

U.S. Patent 4,496,689 discloses a covalently attached complex of alpha-1-proteinase inhibitor with a polymer such as PEG or methoxypolyethylene glycols.

2

U.S. Patent 3,619,371 discloses a polymeric matrix having a biologically active substance chemically bound thereto.

U.S. Patent 3,788,948 discloses use of organic cyanate compounds to bind proteins to polymers.

U.S. Patent 3,876,501 discloses activation of water-soluble carbohydrates with cyanogen bromide to improve their binding to enzymes and other proteins.

U.S. Patent 4,055,635 discloses pharmaceutical compositions of a proteolytic enzyme linked covalently to a polymeric substance.

EP 152,847 discloses an enzyme conjugate composition comprising an enzyme conjugate, a calcium salt, and a polyethylene glycol.

JP 57192435 published November 26, 1982 discloses modified polypeptides where all or part of the amino groups are substituted with a polyethoxyl moiety. DE 2312615 published September 27, 1973 discloses coupling of polymers to compounds containing hydroxy or amino groups.

EP 147,761 discloses a covalent conjugate of alpha-1-proteinase inhibitor and water-soluble polymer, where the polymer may be polyethylene glycol.

U.S. Patent No. 4,414,147 describes rendering interferon less hydrophobic by conjugating it to an anhydride of a dicarboxylic acid such as poly(ethylene succinic anhydride).

EP 154,316, published September 11, 1985 to Takeda Chemical Industries, Ltd., discloses chemically modified lymphokines containing PEG bonded directly to at least one primary amino group of a lymphokine such as alpha, beta or gamma interferon or interleukin-2.

In addition to these patents and patent publications, several articles discuss the concept of using activated PEG or PPG as a modifying agent for proteins such as enzymes, IgG and albumin. For example, Inada et al., Biochem and Biophys. Res. Comm., 122, 845-850 (1984) disclose modifying water-soluble lipoprotein lipase to make it soluble in organic solvents such as benzene by using cyanuric chloride as a coupling agent with PEG. Takahashi et al., Biochem. and Biophys. Res. Comm., 121, 261-265 (1984) disclose modifying horseradish peroxidase using cyanuric chloride triazine with PEG to make the water-soluble enzyme active and soluble in benzene. Suzuki et al., Biochem. Biophys. Acta, 788, 248-255 (1984) disclose suppression of aggregation of IgG using cyanuric chloride activated PEG. Abuchowski et al., Cancer Biochem. Biophys., 7, 175-186 (1984) state that modification of asparaginases from E. coli and Vibrio succinogenes using PEG activated by succinimidyl succinate increases the half-life and decreases the immunogenicity of the proteins. Davis et al., Biomedical Polymers, (New York: Academic Press, 1980), p. 441-451 disclose that enzymes normally insoluble may be solubilized by PEG attachment without further details. Several other articles discuss modification of enzymes such as uricase, streptokinase, catalase, arginase and asparaginase with PEG activated by succinimidyl succinate or cyanuric chloride to increase half-life and decrease the immunogenicity of the protein.

None of these references, however, disclose how to use a polymer modification process to improve the biological activity and pharmacokinetics of tumor necrosis factor (TNF).

Accordingly, the present invention provides for modifying tumor necrosis factor to retain its biological activity and potentially to increase the physiological half-life thereof and decrease immunogenicity by reducing or eliminating aggregation of the protein or by masking antigenic determinants. It is also expected that the prolonged half-life of the modified TNF herein is related to the efficacy of the protein.

More specifically, the present invention is directed to a pharmaceutical composition comprising a non-toxic, inert, pharmaceutically acceptable aqueous carrier medium in which is dissolved a biologically active selectively conjugated tumor necrosis factor (TNF) wherein the TNF is covalently conjugated to a water-soluble polymer selected from the group consisting of polyethylene glycol polymers and polyoxyethylated polyols, said polyethylene glycol polymer being unsubstituted or substituted at one end with an alkyl group and said polyol being unsubstituted, and wherein the selectively conjugated TNF contains approximately equal amounts of unmodified TNF monomers and polymer-conjugated TNF monomers as determined by SDS-PAGE.

Preferably the polymer is unsubstituted polyethylene glycol (PEG) or monomethyl PEG (mPEG), and it is coupled to the TNF via an amide linkage formed from the 4-hydroxy-3-nitrobenzene sulfonate ester or the N-hydroxysuccinimide ester of a PEG or mPEG carboxylic acid.

Another aspect of this invention resides in a process for preparing a pharmaceutical composition comprising:

(a) preparing a water-soluble polymer with at least one terminal reactive group, where the polymer is selected from the group consisting of polyethylene glycol polymers and polyoxyethylated polyols, wherein said polyethylene glycol polymer is unsubstituted or substituted at one end with an alkyl group and said polyol is unsubstituted;

(b) reacting biologically active tumor necrosis factor (TNF) with the reactive group of said polymer so as to provide a biologically active, selectively conjugated TNF which contains approximately equal amounts of unmodified TNF monomers and polymer-conjugated TNF monomers as determined by SDS-PAGE; and

(c) formulating said TNF in a non-toxic, inert, pharmaceutically acceptable aqueous carrier medium.

The TNF herein may be obtained from tissue cultures or by recombinant techniques, and from any mammalian source such as, eg, mouse, rat, rabbit, primate, pig and human. Preferably such protein is derived from a human source, and more preferably is recombinant human TNF.

The human TNF herein may be obtained in recombinant form as described by Pennica et al, Nature, (1984) 312:724-729; Yamada et al, J. Biotechnology, (1985) 3:141-153; Wang et al, Science (1985), 228:149-154; Shirai et al, Nature (London), (1985) 313:803-806; EP 155,549 published September 29, 1985; EP 158,286 published October 16, 1985; EP 168,214 published January 15, 1986; and PCT US85/01921 published April 24, 1986. Recombinant rabbit TNF may be obtained as described in EP 146,026 published June 26, 1985 and EP 148,311 published July 17, 1985.

The cloning of human TNF having 151 and 155 amino acids (2 and 6 less than the native form) is disclosed in EP 155,549, published September 25, 1985 (Dainippon Pharmaceutical Co Ltd), and human TNF having 155 amino acids is disclosed in EP 158,286, published October 16, 1985 (Asahi Kasei Kogyo Kabushiki Kaisha) and corresponding GB 2,158,829A, published November 20, 1985. The cloning of mature TNF (157 amino acids) and various modified forms (muteins) thereof is disclosed in EP 168,214, published January 15, 1986 (Genentech) and PCT US85/01921, filed October 3, 1985, published April 24, 1986 (Cetus Corporation).

Preferably, the TNF herein is a human TNF mutein wherein the first eight amino acid residues have been deleted, using the procedure described in PCT WO86/02381 or the TNF is a cysteine-depleted mutein prepared similarly to that described in US Patent No 4,518,584, supra.

The precise chemical structure of the TNF herein will depend on a number of factors. As ionizable amino and carboxyl groups are present in the molecule, a particular form of TNF may be obtained as an acidic or basic salt, or in neutral form. All such preparations which retain their bioactivity when placed in suitable environmental conditions are included in the definition of TNF herein. Further, the primary amino acid sequence of the TNF may be augmented by derivatization using sugar moieties (glycosylation) or by other supplementary molecules such as lipids, phosphate and acetyl groups, more commonly by conjugation with saccharides. Certain aspects of such augmentation are accomplished through post-translational processing systems of the producing host; other such modifications may be introduced in vitro. In any event, such modifications are included in the definition of TNF herein so long as the bioactivity of the TNF is not destroyed. It is expected, of course, that such modifications may quantitatively or qualitatively affect the bioactivity by either enhancing or diminishing the activity of the TNF in the various assays.

The term "selectively conjugated" as used herein to apply to the TNF refers to TNF which is covalently bonded via one of its amino acid residues. While the residues may be any reactive amino acids on the protein, such as one or two cysteines or the N-terminal amino acid group, preferably the reactive amino acid is lysine, which is linked to the reactive group of the activated polymer through its free $\epsilon$-amino group, or glutamic or aspartic acid, which is linked to the polymer through an amide bond.

The polymer to which the TNF is attached is a homopolymer or copolymer of polyethylene glycol (PEG) or is a polyoxyethylated polyol, provided in all cases that the polymer is soluble in water at room temperature. Examples of copolymers include, e.g., copolymers of ethylene glycol and propylene glycol, or of ethylene and maleic anhydride. Examples of polyoxyethylated polyols include, for example, polyoxyethylated glycerol, polyoxyethylated sorbitol a polyoxyethylated glucose. Preferably, the PEG polymer is a homopolymer and the polyoxyethylated polyol is polyoxyethylated glycerol.

The glycerol backbone of polyoxyethylated glycerol is the same backbone occurring naturally in, for example, animals and humans as mono-, di- and triglycerides. Therefore, this branching would not necessarily be seen as a foreign agent in the body.

The polymer need not have any particular molecular weight, but it is preferred that the molecular weight be between about 300 and 100,000, more preferably between 350 and 40,000, depending, for example, on the particular protein employed.

Preferably, the PEG polymer is unsubstituted, but it may also be substituted at one end with an alkyl group. Preferably, the alkyl group is a $C_1$-$C_4$ alkyl group, and most preferably a methyl group. Most preferably, the polymer is an unsubstituted homopolymer of PEG or a monomethyl-substituted homopolymer of PEG, and has a molecular weight of about 350 to 40,000.

The TNF is conjugated via a terminal reactive group on the polymer. The polymer with the reactive group(s) is designated herein as activated polymer. The reactive group selectively reacts with free amino or other reactive groups on the TNF. As reaction of the reactive group with too many active groups on TNF may not be possible to avoid completely, it is recommended that generally from about 1 to 10 moles, preferably 1-5 moles, of activated polymer per mole of TNF, depending on the TNF concentration, is employed. The ultimate amount employed is a balance to maintain optimum activity, while at the same time optimizing, if possible, the half-life of the protein. Preferably, at least about 10%, more preferably 25%, more preferably 50% and most preferably 100%, of the biological activity of the protein is retained.

The covalent modification reaction may take place by any suitable method generally used for reacting biologically active materials with inert polymers, preferably at about pH 5-9, if the reactive groups on the TNF are lysine groups. Generally the process involves preparing an activated polymer (with at least one terminal hydroxyl group) and thereafter reacting the TNF with the activated polymer to produce the modified TNF suitable for formulation.

The above modification reaction can be performed by several methods, which may involve one or more steps. Examples of suitable modifying agents which can be used to produce the activated polymer in a one-step reaction include cyanuric acid chloride (2,4,6-trichloro-S-triazine) and cyanuric acid fluoride.

In a preferred embodiment the modification reaction takes place in two steps wherein the polymer is reacted first with an acid anhydride such as succinic or glutaric anhydride to form a carboxylic acid, and the carboxylic acid is then reacted with a compound capable of reacting with the carboxylic acid to form an activated polymer with a reactive ester group which is capable of reacting with the protein. Examples of such compounds include N-hydroxysuccinimide and 4-hydroxy-3-nitrobenzene sulfonic acid, and preferably N-hydroxysuccinimide or 4-hydroxy-3-nitrobenzene sulfonic acid is used. For example, monomethyl substituted PEG may be reacted at elevated temperatures, preferably about 100-110°C for four hours, with glutaric anhydride. The monomethyl PEG-glutaric acid thus produced is then reacted with N-hydroxysuccinimide in the presence of a carbodiimide reagent such as dicyclohexyl or isopropyl carbodiimide to produce the activated polymer, methoxypolyethylene glycolyl-N-succinimidyl glutarate, which can then be reacted with the TNF. This method is described in detail in Abuchowski et al., Cancer Biochem. Biophys., 7, 175-186 (1984). In another example the monomethyl substituted PEG may be reacted with glutaric anhydride followed by reaction with 4-hydroxy-3-nitrobenzene sulfonic acid (HNSA) in the presence of dicyclohexyl carbodiimide to produce the activated polymer. HNSA is described in Bhatnagar et al., Peptides: Synthesis-Structure-Function, Proceedings of the Seventh American Peptide Symposium, Rich, et al. (eds.) (Pierce Chemical Co., Rockford IL, 1981), p. 97-100, and in Nitecki et al., High-Technology Route to Virus Vaccines (American Society for Microbiology: 1986) entitled "Novel Agent for Coupling Synthetic Peptides to Carriers and Its Application."

As ester bonds are chemically and physiologically less stable than amide bonds, it may be preferable to use chemical transformations in the coupling reaction which would produce carboxylic acids or amides without concurrent production of esters.

The TNF thus modified is then formulated in a non-toxic, inert, pharmaceutically acceptable aqueous carrier medium, preferably at a pH of about 3 to 8, more preferably 6-8. For in vitro applications, the modes of administration and formulation are not critical. Aqueous formulations compatible with the culture or perfusion medium will generally be used. When used in vivo for therapy, the sterile product will consist of a mixture of TNF dissolved in an aqueous buffer in an amount which will provide a pharmaceutically acceptable pH when the mixture is reconstituted. A water-soluble carrier such as mannitol may optionally be added to the medium.

The dosage level of protein in the formulation will depend on the in vivo efficacy data obtained after preclinical testing and will depend mainly on the ultimate use.

If the formulation is lyophilized, the lyophilized mixture may be reconstituted by injecting into the vial a conventional parenteral aqueous injection such as, e.g., distilled water.

The reconstituted formulation prepared as described above is suitable for parenteral administration to humans or other mammals in therapeutically effective amounts (i.e., amounts which eliminate or reduce the patient's pathological condition) to provide therapy thereto, i.e., to kill neoplastic cells.

The dose and dosage regimen of the TNF will depend, for example, on the pharmacokinetics of the drug, the nature of the disease, the characteristics of the TNF, the patient, and the patient's history. For example, different modified TNF proteins are expected to have different pharmacokinetic and therapeutic properties which are advantageous for different routes of administration. A long-acting drug might only be administered every 3-4 days every week, or once every two weeks. The clearance rate can be varied to give ultimate flexibility to fit the particular need of the patient by changing, e.g., the type of polymer and size of polymer attached.

In the following examples, which illustrate the invention further, all parts and percentages are by weight unless otherwise noted, and all temperatures are in degrees Celsius.

EXAMPLE I

Preparation of PEGylated TNF

A. Preparation of PEG-Ester

A linear, monomethyl substituted ester of PEG of molecular weight 5000 was obtained by first reacting monomethyl PEG-5000, which is commercially available, with glutaric anhydride at 100 to 110°C for four hours or by a method similar to that of Abuchowski et al., Cancer Biochem. Biophys., 7, 175-186 (1984). The resulting PEG-glutaric was reacted with N-hydroxysuccinimide in the presence of dicyclohexylcarbodiimide, as described in detail by Abuchowski et al., supra, on page 176. The resulting product is monomethoxypolyethylene glycolyl N-succinimidyl glutarate, hereinafter designated as PEG*.

In an alternative step, and by a similar method, monomethoxypolyethylene glycolyl N-succinimidyl succinate may be prepared using succinic anhydride in place of glutaric anhydride. In a third and similar method, a PEG carboxylic ester-HNSA may be prepared using HNSA in place of N-hydroxysuccinimide. This latter ester preparation is described by Bhatnagar et al., supra and by Nitecki et al., supra. The PEG carboxylic ester-HNSA may be used as the activated PEG in the procedures described in this example.

B. Coupling of PEG* to TNF

A mutein of human TNF having the first eight amino acids deleted from the N-terminus was prepared as follows. TNF was induced from publicly available HL-60 cells and purified and sequenced. Then an intronless sequence encoding human TNF was prepared by producing enriched mRNA, constructing a cDNA library, selecting a probe, and probing the library to recover the sequence. Then an ATG start codon was introduced immediately preceding the GTC sequence encoding N-terminal valine of the mature protein by site-directed mutagenesis. Clones were selected and strands ligated into expression vectors to obtain procaryotic expression of the mutein. The mutein was then purified by column purification using any standard purification technique and recovered in the purification buffer.

To 1 mg of the TNF mutein in 0.5 ml of 50 mM sodium phosphate, pH 8.2, was added freshly prepared aqueous PEG* in molar ratios from 1 to 50 per mole of TNF and sufficient 50 mM borate buffer, pH 9.0, to give a final volume of 1.0 ml. The PEG* was dissolved at 100 mg/ml in cold 10 mM sodium phosphate, pH 7.0. After thorough mixing the reaction proceeded at 23°C for 30 minutes. The reaction was finished in 30 minutes and the PEG*-TNF was ready for further processing.

C. Purification of Modified TNF

Using DEAE ion exchange chromatography, the various forms of PEGylated TNF were separated from unmodified TNF. A sample of 1 mg of TNF modified with a five-fold molar excess of PEG* was applied to the column. A linear gradient of 0-200 mM sodium chloride in 30 minutes was run at 1.0 ml/min. on a 7.5 mm × 7.5 cm TSK DEAE-5-PW column. The buffer was 10 mM Tris chloride pH 8.2. Aliquots of the fractions were assayed for TNF bioactivity as described in Example II.B.

EXAMPLE II

Characterization of PEGylated TNF

A. Size characterization of modified TNF products

The fractions from the DEAE column were analyzed on a SDS-PAGE (15%, non-reducing) gel. Before being applied to the gel the fractions were mixed with concentrated SDS sample buffer. Then 2.5 micrograms of protein were loaded onto a gel lane and electrophoresed for about 1.5 hours at 35 milliAmps. The gels were stained and then destained.

The latest eluting peak from the ion exchange column appeared to be unmodified TNF by SDS-PAGE. It consisted of the monomer TNF band at 15,000 molecular weight. The earlier eluting peaks from ion exchange contained a certain proportion of higher molecular weight bands, seen on SDS-PAGE. These bands were the TNF monomer conjugated with one, two or more molecules of PEG*. The relative proportion of unmodified TNF monomer to the modified monomers decreased as the peaks eluted earlier from the column. That is, the earlier eluting peaks were more highly modified with PEG. The proportion of TNF

EP 0 247 860 B1

modified with two and three molecules of PEG* was also highest in the early eluting peaks.

B. Bioactivity of PEGylated TNF as a Function of the Extent of Modification

Fractions from the aforementioned DEAE column separation of TNF modified with a five-fold molar excess of PEG* were assayed by the L-929 TNF cytotoxic bioassay described below.

The L-929 assay system is an improved convenient in vitro assay which permits rapid measurement of TNF activity. Its degree of correlation with the in vivo tumor necrosis assay of Carswell, et al, Proc. Natl. Acad. Sci (USA) (1975), 72:3666, supra, is, at present, unknown; however, as it utilizes murine tumor cells specifically, the correlation is expected to be high. The protein designated lymphotoxin in EPO Publication No. 0100641 also gives activity in this assay. The assay is similar in concept to that disclosed in U.S. Patent 4,457,916 which used murine L-M cells and methylene blue staining. However, the L-929 assay has been shown herein to correlate (for HL-60-derived TNF) with human tumor cell line cytotoxicity.

In the L-929 assay system, L-929 cells are prepared overnight as monolayers in microtiter plates. The test samples are diluted two-fold across the plate, UV irradiated, and then added onto the prepared cell monolayers. The culture media in the wells are then brought to 1 $\mu$g/ml actinomycin D. The plates are allowed to incubate 18 hours at 37°C and the plates are scored visually under the microscope. Each well is given a 25, 50, 75 or 100% mark signifying the extent of cell death in the well. One unit of TNF activity is defined as the reciprocal of the dilution at which 50% killing occurs.

The results of the L-929 assays of the DEAE column fractions are shown in Table I. Fractions 48-58 are modified TNF and Fraction 60 is unmodified TNF. The results suggest that the PEGylated TNF in Fractions 56-58 has the same specific activity as the unmodified TNF. More highly PEGylated forms of TNF (Fractions 48-54) show decreased specific activity. The active PEG-TNF contains approximately equal amounts of unmodified TNF monomers and mono-PEGylated monomers as determined by SDS-PAGE. As TNF is a dimer, most likely the active species is a mono-PEG-TNF monomer combined with an unmodified monomer. The combination of two modified monomers (Fractions 52-54) is almost 2-10-fold less active than the unmodified TNF.

## TABLE I
### Bioactivity of PEGylated TNF

| Fraction From Column | Bioactivity (Units/mg TNF) |
|---|---|
| 48 | $1.30 \times 10^6$ |
| 50 | $1.26 \times 10^6$ |
| 52 | $2.04 \times 10^6$ |
| 54 | $5.88 \times 10^6$ |
| 56 | $1.32 \times 10^7$ |
| 58 | $1.12 \times 10^7$ |
| 60 | $1.47 \times 10^7$ |

When the reaction was carried out as described in Example I using 5 moles of PEG* per mole of TNF, the unfractionated TNF had a specific activity of 20% of the unmodified TNF; at a 10-fold excess of PEG* there was 1% of the specific activity remaining.

C. Pharmacokinetics of PEGylated TNF compared to unmodified TNF

It is expected that the active TNF species has a longer half-life than the unmodified TNF when injected into mice.

EXAMPLE III

Preparation of PEGylated TNF With 2000 Molecular Weight PEG

7

A TNF derivative of PEG of molecular weight 2000 was prepared generally by the method described in Example I using PEG-2000. The resulting derivative was biologically active.

EXAMPLE IV

Preparation of TNF Modified With Polyoxyethylated Glycerol (POG) (Expected Results)

A. Preparation of Activated POG-TNF

Polyoxyethylated glycerol (POG) of molecular weight 5000 was custom synthesized by Polysciences.

To 10 g of POG was added 2.28 g glutaric anhydride (a tenfold excess over POG). The mixture was stirred for two hours at 110°C and cooled. This was dissolved in 20 ml CHCl$_3$ and poured slowly into 500 ml ether with vigorous stirring. The product was collected and rinsed with ether to yield about 90% POG glutarate product. This product was reacted with N-hydroxysuccinimide as described in Example I.A. to yield the active ester POG-glutaryl N-hydroxysuccinimide (POG*). It is expected that the TNF described in Example I.B. may be reacted with the POG* at room temperature under the conditions generally described in Example I.B. The reaction mixture may then be applied to a separation column for purification and the fractions analyzed for homogeneity by SDS-PAGE and for bioactivity as described in Example II.B.

In summary, the present invention is seen to provide a pharmaceutical composition wherein biologically active TNF protein is selectively conjugated to a polymer of PEG or of a polyoxyethylated polyol.

**Claims**

1. A pharmaceutical composition comprising a non-toxic, inert, pharmaceutically acceptable aqueous carrier medium in which is dissolved a biologically active selectively conjugated tumor necrosis factor (TNF) wherein the TNF is covalently conjugated to a water-soluble polymer selected from polyethylene glycol polymers and polyoxyethylated polyols, said polyethylene glycol polymer being unsubstituted or substituted at one end with an alkyl group and said polyol being unsubstituted, and wherein the selectively conjugated TNF contains approximately equal amounts of unmodified TNF monomers and polymer-conjugated TNF monomers as determined by SDS-PAGE.

2. A composition according to claim 1 wherein said polymer is a homopolymer and has a molecular weight of about 300 to 100,000.

3. A composition according to claim 1 or 2 wherein said polymer is conjugated to the TNF via the 4-hydroxy-3-nitrobenzene sulfonate ester or the N-hydroxysuccinimide ester of a carboxylic acid of said polymer.

4. A composition according to any one of claims 1-3 wherein said polymer is either a homopolymer selected from unsubstituted polyethylene glycol or monomethyl polyethylene glycol, or a polyoxyethylated glycerol, and said TNF is human recombinant TNF or a mutein thereof.

5. A process for preparing a pharmaceutical composition comprising:
(a) preparing a water-soluble polymer having at least one terminal reactive group where said polymer is selected from polyethylene glycol polymers and polyoxyethylated polyols, wherein said polyethylene glycol polymer is unsubstituted or substituted at one end with an alkyl group, and said polyol is unsubstituted;
(b) reacting biologically active tumor necrosis factor (TNF) with the reactive group of said polymer so as to provide a biologically active, selectively conjugated TNF which contains approximately equal amounts of unmodified TNF monomers and polymer-conjugated TNF monomers as determined by SDS-PAGE;
(c) formulating said TNF in a non-toxic, inert, pharmaceutically acceptable aqueous carrier medium.

6. A process according to claim 5 wherein said polymer has a molecular weight of about 300 to 100,000 and said TNF is formulated at pH 6-8.

7. A process according to claim 5 or 6 wherein step (a) comprises reacting the polymer with an acid anhydride to form a carboxylic acid, and reacting the carboxylic acid with a compound capable of reacting with the acid to form said activated polymer with a reactive ester group.

8. A process according to claim 7 wherein the acid anhydride is glutaric anhydride and the compound capable of reacting with the acid in the presence of a carbodiimide is N-hydroxysuccinimide or 4-hydroxy-3-nitrobenzene sulfonic acid.

9. A process according to claim 8 wherein the reactive group reacts with one lysine residue of said TNF and step (b) is carried out at a pH of about 8-10.

10. A process according to any one of claims 5-9 wherein said TNF is human recombinant TNF or a mutein thereof and said polymer is either a homopolymer selected from unsubstituted polyethylene glycol or monomethyl polyethylene glycol, or a polyoxyethylated glycerol.

**Revendications**

1. Composition pharmaceutique comprenant un milieu aqueux formant véhicule, non toxique, inerte et pharmaceutiquement acceptable, dans lequel est dissous un facteur de nécrose tumorale (TNF) sélectivement conjugué et biologiquement actif, le TNF étant conjugué de manière covalente avec un polymère soluble dans l'eau choisi parmi les polyéthylène glycols et les polyols polyoxyéthylés, ce polyéthylène glycol étant non substitué ou substitué à une extrémité avec un groupe alkyle, et ce polyol étant non substitué, et dans laquelle le TNF sélectivement conjugué, contient des quantités à peu près égales de monomères TNF non modifiés et de monomères TNF conjugués avec un polymère, ainsi que cela est déterminé par SDS-PAGE.

2. Composition selon la revendication 1, dans laquelle le polymère est un homopolymère, et a un poids moléculaire d'environ 300 à 100 000.

3. Composition selon la revendication 1 ou 2, dans laquelle le polymère est conjugué avec le TNF, par l'intermédiaire du 4-hydroxy-3-nibrobenzène sulfonate ester ou de l'ester d'un acide carboxylique dérivé de ce polymère, avec le N-hydroxysuccinimide.

4. Composition selon l'une quelconque des revendications 1 à 3, dans laquelle le polymère est un homopolymère choisi parmi un polyéthylène glycol non substitué ou un monométhyl polyéthylène glycol, ou un glycérol polyoxyéthylé, et dans laquelle ce TNF est un TNF humain recombinant ou une mutéine de celui-ci.

5. Procédé de préparation d'une composition pharmaceutique, selon lequel :
   (a) on prépare un polymère soluble dans l'eau comportant au moins un groupe terminal réactif, ce polymère étant choisi parmi les polyéthylène glycols et les polyols polyoxyéthylés, le polyéthylène glycol étant non substitué ou substitué à une extrémité avec un groupe alkyle, et ce polyol étant non substitué ;
   (b) on fait réagir un facteur de nécrose tumorale (TNF) biologiquement actif, avec le groupe réactif de ce polymère, de façon à procurer un TNF sélectivement conjugué et biologiquement actif, contenant des quantités à peu près égales de monomères TNF non modifiés et de monomères TNF conjugués avec un polymère, ainsi que cela est déterminé par SDS-PAGE ;
   (c) on incorpore ce TNF dans un milieu aqueux formant véhicule non toxique, inerte et pharmaceutiquement acceptable.

6. Procédé selon la revendication 5, dans lequel ce polymère a un poids moléculaire d'environ 300 à 100 000, et le TNF est incorporé dans une composition à un pH de 6 à 8.

7. Procédé selon la revendication 5 ou 6, dans lequel, dans l'étape (a), on fait réagir le polymère avec un anhydride d'acide, pour former un acide carboxylique, et on fait réagir l'acide carboxylique avec un composé capable de réagir avec l'acide pour former ce polymère activé avec un groupe ester réactif.

8. Procédé selon la revendication 7, dans lequel l'anhydride d'acide est l'anhydride glutarique, et le composé capable de réagir avec l'acide en présence d'un carbodiimide, est le N-hydroxysuccinimide ou l'acide 4-hydroxy-3-nitrobenzène sulfonique.

9

**9.** Procédé selon la revendication 8, dans lequel le groupe réactif réagit avec au moins un résidu lysine du TNF, et dans lequel l'étape (b) est effectuée à un pH d'environ 8 à 10.

**10.** Procédé selon l'une quelconque des revendications 7 à 9, dans lequel le TNF est un TNF humain recombinant ou une mutéine de celui-ci, et le polymère est un homopolymère choisi parmi un polyéthylène glycol non substitué ou un monométhyl polyéthylène glycol, ou un glycérol polyoxyéthylé.

**Patentansprüche**

**1.** Arzneimittel, umfassend ein nicht-toxisches, inertes, pharmazeutisch verträgliches wäßriges Trägermedium, in dem ein biologisch aktiver, selektiv konjugierter Tumornekrosefaktor (TNF) gelöst ist, wobei der TNF kovalent mit einem wasserlöslichen Polymer konjugiert ist, das ausgewählt ist aus Polyethylenglykolpolymeren und polyoxyethylierten Polyolen, wobei das Polyethylenglykolpolymer unsubstituiert ist oder an einem Ende mit einem Alkylrest substituiert ist und das Polyol unsubstituiert ist, und wobei der selektiv konjugierte TNF etwa gleiche Mengen von unmodifizierten TNF-Monomeren und polymerkonjugierten TNF-Monomeren, wie durch SDS-PAGE bestimmt, enthält.

**2.** Mittel nach Anspruch 1, wobei das Polymer ein Homopolymer ist und ein Molekulargewicht von etwa 300 bis 100 000 aufweist.

**3.** Mittel nach Anspruch 1 oder 2, wobei das Polymer mit dem TNF über den 4-Hydroxy-3-nitrobenzolsulfonatester oder den N-Hydroxysuccinimidester einer Carbonsäure des Polymers konjugiert ist.

**4.** Mittel nach einem der Ansprüche 1 bis 3, wobei das Polymer entweder ein Homopolymer ist ausgewählt aus unsubstituiertem Polyethylenglykol oder Monomethylpolyethylenglykol, oder ein polyoxyethyliertes Glycerin, und wobei der TNF ein menschlicher rekombinanter TNF oder ein Mutein davon ist.

**5.** Verfahren zur Herstellung eines Arzneimittels, umfassend:
(a) Herstellen eines wasserlöslichen Polymers mit mindestens einer endständigen reaktiven Gruppe, wobei das Polymer ausgewählt ist aus Polyethylenglykolpolymeren und polyoxyethylierten Polyolen, wobei das Polyethylenglykolpolymer unsubstituiert ist oder an einem Ende mit einem Alkylrest substituiert ist und das Polyol unsubstituiert ist;
(b) Umsetzen eines biologisch aktiven Tumornekrosefaktors (TNF) mit der reaktiven Gruppe des Polymers, wodurch ein biologisch aktiver, selektiv konjugiertes TNF bereitgestellt wird, der etwa gleiche Mengen von unmodifizierten TNF-Monomeren und Polymer-konjugierten TNF-Monomeren, wie durch SDS-PAGE bestimmt, enthält;
(c) Formulieren des TNF in einem nicht-toxischen, inerten, pharmazeutisch verträglichen wäßrigen Trägermedium.

**6.** Verfahren nach Anspruch 5, wobei das Polymer ein Molekulargewicht von etwa 300 bis 100 000 aufweist und der TNF bei einem pH-Wert von 6 bis 8 formuliert wird.

**7.** Verfahren nach Anspruch 5 oder 6, wobei Schritt (a) die Umsetzung des Polymers mit einem Säureanhydrid zur Herstellung einer Carbonsäure und die Umsetzung der Carbonsäure mit einer Verbindung, die mit der Säure zu dem aktivierten Polymer mit einer reaktiven Estergruppe reagieren kann, umfaßt.

**8.** Verfahren nach Anspruch 7, wobei das Säureanhydrid Glutarsäureanhydrid ist und die Verbindung, die mit der Säure in Gegenwart eines Carbodiimids reagieren kann, N-Hydroxysuccinimid oder 4-Hydroxy-3-nitrobenzolsulfonsäure ist.

**9.** Verfahren nach Anspruch 8, wobei die reaktive Gruppe mit einem Lysinrest des TNF reagiert und Schritt (b) bei einem pH-Wert von etwa 8 bis 10 durchgeführt wird.

**10.** Verfahren nach einem der Ansprüche 5 bis 9, wobei der TNF ein menschlicher rekombinanter TNF oder ein Mutein davon ist, und das Polymer entweder ein Homopolymer, ausgewählt aus unsubstituiertem Polyethylenglykol oder Monomethylpolyethylenglykol, oder ein polyoxyethyliertes Glycerin ist.